# EUROPEAN PATENT SPECIFICATION

(11) **EP 3 116 470 B1**
(45) Date of publication and mention of the grant of the patent: **08.05.2019**
(21) Application number: 15770631.8
(22) Date of filing: 25.02.2015
(51) Int. Cl.: A61K 8/34, A61K 8/41, A61Q 19/00

(54) **COSMETIC COMPOSITIONS OF MITOCHONDRIALLY TARGETED ANTIOXIDANTS**
KOSMETISCHE ZUSAMMENSETZUNGEN AUS AUF MITOCHONDRIEN GERICHTETEN ANTIOXIDANZIEN
COMPOSITIONS COSMÉTIQUES D'ANTIOXYDANTS À CIBLE MITOCHONDRIALE

(30) Priority: 25.02.2014 US 201461944205 P
(43) Date of publication of application: 18.01.2017
(73) Proprietor: Mitotech SA, 2661 Luxembourg (LU)
(72) Inventor: PELEKH, Valeriya Vladimirovna, Moscow 117342 (RU); LYANDIN, Valeriy Vasilievech, Moscow 117437 (RU); SHIBAEVA, Anna Vladimirovna, Puskino Moscow Region 141207 (RU); SKULACHEV, Maxim Vladimirovich, Moscow 119234 (RU); FRIEDHOLL, Lawrence T., River Vale, NJ 07675 (US)
(74) Representative: Adam, Holger
(86) International application number: PCT/IB2015/001030
(87) International publication number: WO 2015/162494

(56) References cited:
- WO-A1-2007/046729
- WO-A1-2012/167236
- US-A1- 2010 292 625
- US-A1- 2012 094 962
- DATABASE GNPD [Online] MINTEL; 1 May 2013 (2013-05-01), "N.M.F. Aquaring Gel Eyefill Patch", XP002746535, Database accession no. 2069885
- DATABASE GNPD [Online] MINTEL; 1 November 2002 (2002-11-01), "Jelly Eyecolor", XP002746536, Database accession no. 178548
- DATABASE GNPD [Online] MINTEL; 1 August 2013 (2013-08-01), "Serum", XP002746537, Database accession no. 2124072
- SKULACHEV V P: "A biochemical approach to the problem of aging: Megaproject on membrane-penetrating ions. The first results and prospects", BIOCHEMISTRY, MAIK NAUKA - INTERPERIODICA, RU, vol. 72, no. 12, 1 December 2007 (2007-12-01), pages 1385-1396, XP009167606, ISSN: 0006-2979
- D A Tsarev ET AL: "EVALUATING THE STABILITY OF A CATIONIC PLASTOQUINONE DERIVATIVE (PDTP) IN VISOMITIN EYE DROPS Translated from Khimiko-Farmatsevticheskii", , 4 April 2013 (2013-04-04), pages 40-44, XP55220497, Retrieved from the Internet: URL:http://rd.springer.com/content/pdf/10. 1007/s11094-013-0932-3.pdf [retrieved on 2015-10-13]
- "The Art of Chemistry", INTERNET CITATION, November 2011 (2011-11), pages 1-35, XP007923236, Retrieved from the Internet: URL:http://ar-pol.com/images/akott/2_AKOTT %20BIOGENICO%20LINE_NOV2011.pdf [retrieved on 2015-10-15]

## Description

### BACKGROUND OF THE INVENTION

### Field of the invention

This invention relates to biology and medicine and in particular to cosmetology, and may be used for manufacturing a composition for protecting the skin from damage caused by free radicals and/or active forms of oxygen.

### Summary of the related art

Mitochondrially targeted antioxidants (MTA) are one of the most promising classes of anti-age products for use in various products including cosmetic products. However, various MTAs, including those in the SkQ class, in multi-component liquid mixtures are not greatly stable over long periods of storage (over 30 days). WO2007/046729 discloses a method of acting on an organism via targeted delivery of antioxidant substances to mitochondria at the expense of the energy of the electrochemical potential of hydrogen atoms. These substances are known as Skulachev ions. US2010/029625 discloses the use of MTAs, including SkQ1 for the acceleration of wound and other body surface injuries healing processes.

The prior art refers to a number of versions of stable compositions of MTA (see for example application WO2012167236), but the number of compositions discovered in technical terms is extremely limited for application as a finished cosmetic product. Therefore, the creation of a finished cosmetic product containing a MTA, and particularly SkQ-class compounds, with sufficient storage stability is an unresolved technical task.

US 2012/0094962 A1 relates to the manufacturing and use of pharmaceutical compositions of medicines (ophthalmic preparations) comprising a mitochondria-adressed antioxidant and a set of auxiliary substances providing effective treatment for ophthalmological diseases in humans and animals. WO 2012/167236 A1 provides stable liquid and solid formulations of oxidized and reduced mitochondria-targeted antioxidants, and methods of their preparation and use.

WO 2007/046729 A1 relates to biology and medicine and, in particular, can be used in medicine to make a pharmaceutical composition for targeted delivery of biologically active substances into mitochondria, driven by proton electro-chemical potential in the mitochondria.

US 2010/0292625 A1 can be used in medicine for preparing a pharmacological composition used for accelerated healing of wounds and the damages by means of addressed (directed) delivery of biologically active agents to mitochondria by means electrochemical potential of hydrogen ions contained therein.

### Definitions

***1.*** ***Mitochondrial antioxidant*** - compound with general formula 1: 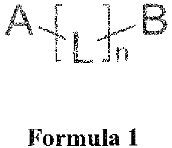
   Where **A is an antioxidant** with general formula 2: 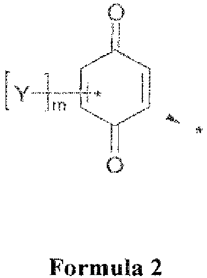 Where Y where m is an integer from 1 to 3; Y refers to single or various substitutes that constitute a lower alkyl or lower alkoxy, including the methyl or methoxy groups;
      and/or its restored form
   **L is a linker group, that is:**
      a) a straight or branched hydrocarbon chain, not necessarily replaced with one or more substitutes, and where necessary containing one or more double or triple bonds; or
      b) a natural isoprenoid chain;
   **n is an integer from 1 to 20;**
   **B is a** Skulachev ion Sk:

      Sk⁺Z⁻

      where Sk is a lipophilic cation; Z is a pharmacologically acceptable anion
***2.*** ***Stable composition for cosmetic use*** is a composition within which the formula 1 compound retains sufficient stability over at least 30 days when stored at a temperature of +5°C.
***3.*** ***Emollients*** are substances from various chemical groups, hydrophilic and lipophilic, capable of softening the skin.
***4.*** ***Multifunctional components*** are components of a cosmetic product that fulfil more than one function.

### Names and description of some components of finished cosmetic products and their manufacturers:

### (manufacturers shown in square brackets)

The composition of components is significant, and the manufacturer and trade product may be different. Manufacturers and trade names are shown as proof of possibility of applying the invention and as an indication for qualified specialists of what ingredients (regardless of their trade names and manufacturers) were used in the present invention.

Biocol LG (Ceratonia Siliqua Gum, Pectin, Algin) [Manufacturer: Biogenico Worldwide]. Biocol AX (Pectin, Xanthan Gum) [Biogenico Worldwide].

Makimousse12 (Sodium Polyacrylate Starch) [Daito Kasei].

Methocel 40-202 (Hydroxypropyl Methylcellulose) [DOW].

Nexbase 2006 FG (Hydrogenated Polydecene) [Neste Oil Oyj].

Silsoft 034 (Caprylyl Methicone) [Momentive Performance Materials].

Sensolene (Ethyl hexyl Olivate) [B@TCompany].

Lexfeel 7 (Neopentyl Glycol Diheptanoate) [Inolex].

Biolin/P (Inulin, Alpha-glucan Oligosaccharide) [Gova Ingredients].

Drieline1S (Sorbitol, Yeast Extract) [LucasMeyer cosmetics].

Peptiskin (Arginine/lysine Polypeptide) [Solabia Group].

Gatuline In-Tense (Caprylic/Capric Triglyceride, Spilanthes Acmella Flower Extract) [Gattefosse].

Vegetensor (Pisum Sativum Extract, Sclerotium Gum) [Alban Muller International]. Argireline (Aqua, Acetyl Hexapeptide-8) [Lipotec Group].

Ecoffea (Aqua, Glycerine, Coffea Arabica (Coffee) Seedcake Extract) [Chemyunion Quimica LTDA].

Dermocea (Aqua, Sucrose, Meristotheca Dakarensis Extract, Jania Rubens Extract) [Gelyma]. RonaCare Cyclopeptide-5 (Aqua, Alcohol, Lecithin, Ectoin, Cyclotetrapeptide-24 Aminocyclohexane Carboxylate) [Merck KGaA].

RonaCare Luremin (Dihydroxymethylchromone, Sorbitol) [Merck KGaA].

RonaFlair MTU (Bismuth Oxychloride) [Merck KGaA].

Colorona Oriental Beige (Mica, C177891 (TiO2), Cl77491 (FeO)) [Merck KGaA].

Colorona Red Gold (Mica, Titanium Dioxide, Iron Oxide) [Merck KGaA].

Microcare IT (Methylchloroisothiazolinone, Methylisothiazolinone) [Thor Sp].

Dipotassium Glycyrrhizate [Selco].

Ethyl Ascorbic Acid [Selco].

Allantoin [DSM]

D-Panthenol [DSM]

Sodium Ascorbyl Phosphate. [BASF]

Propylene Glycol. [INEOS].

Hydrolite-5 (Pentylene Glycol) [Symrise].

Sensiva SC 50 (Ethyl hexyl glycerine) [Schulke@Mayr].

### Description of invention:

The present invention relates to a composition comprising a mitochondrial antioxidant SkQ1: 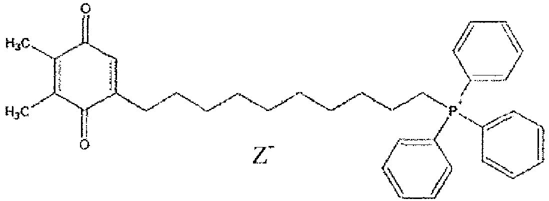 or its reduced form,
where Z⁻ is a pharmacologically acceptable anion,
and ceratonia siliqua gum, algin, pectin, ethyl hexyl glycerine, pentylene glycol, and propylene glycol.

The present invention also relates to a cosmetic composition consisting of a composition according to claim 1 and a further compound selected from the group consisting of benzalkonium chloride, hydrogenated polydecene, caprylyl methicone, ethyl hexyl olivate, D-Panthenol, neopentylglycol diheptanoate, inulin in mixture with a-glucane oligosaccharide, ethyl ascorbic acid, dipotassium glycyrrhizate, allantoin, and sodium ascorbylphosphate.

The present disclosure provides a composition and manufacturing process for finished multi-component cosmetic products containing MTAs. Preferably, one aspect of the invention is stable (cosmetic) composition containing mitochondrial antioxidant, and at least one additional component from the following list: gel-forming component, emollient, multi-functional component with antimicrobial activity, additional component - active cosmetic ingredient.

Examples of the disclosure are the following compositions:

| Component | Concentration |
|---|---|
| **Composition 1** | |
| SkQ1 | 1 nM - 1 mM |
| Gel-forming agent Biocol LG (carob tree gum, pectin, sodium alginate) | 0.2-5% |
| Hydrogenated polydecene | 0.01-40% |
| Caprylyl methicone | 0.01-40% |
| Ethyl hexyl glycerine | 0.1-2% |
| Pentylene glycol | 0.5-10% |
| Propylene glycol | 1-50% |
| Gatuline In-Tense | 0.1-5% |

| **Composition 2** | |
|---|---|
| SkQ1 | 10 mcM |
| Gel-forming agent Biocol LG (carob tree gum, pectin, sodium alginate) | 2.9% |
| Hydrogenated polydecene | 0.5% |
| Caprylyl methicone | 0.5% |
| Ethyl hexyl glycerine | 0.3% |
| Pentylene glycol | 5% |
| Propylene glycol | 3% |
| Gatuline In-Tense | 2% |
| | |

| **Composition 3** | |
|---|---|
| SkQ1 | 10 mcM |
| Gel-forming agent Biocol LG (carob tree gum, pectin, sodium alginate) | 2.9% |
| Hydrogenated polydecene | 0.5% |
| Caprylyl methicone | 0.5% |
| Ethyl hexyl glycerine | 0.3% |
| Pentylene glycol | 5% |
| Propylene glycol | 3% |
| Gatuline In-Tense | 2% |
| Microcare IT (methylchloroisothiazolinone (and) methylisothiazolinone) | 0.1% |

| Composition 4 (on basis of exp. of sample 1) | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Benzalkonium chloride | 0.01% |

| Composition 5 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Hydrogenated polydecene (Nexbase) | 1% |

| Composition 6 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 1% |

| Composition 7 | |
|---|---|
| SkQ1 | 0.1-1,000 mcM |
| Biocol MG | 2.9% |
| Propylene glycol | 3% |
| Pentylene glycol | 5% |
| Ethyl hexyl glycerine | 0.3% |
| D-panthenol | 0.75% |

| Composition 8 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Neopentyl glycol diheptanoate (Lexfeel 7) | 1% |

| Reference Composition 9 | |
|---|---|
| SkQ1 | 0.1-1,000 mcM |
| Sodium Polyacrylate Starch (Makimousse 12) | 1% |
| Propylene glycol | 3% |
| Pentylene glycol | 5% |
| Ethyl hexyl glycerine | 0.3% |

| Composition 10 | (on base of exp. of example 2) |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| Caprylic/Capric triglyceride (and) Spilanthes Acmella Flower Extract (Gatuline In-Tense) | 2% |

| Composition 11 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| Aqua (and) Acetyl Hexapeptide-8 (Argireline) | 5% |

| Composition 12 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| Aqua (and) Glycerine (and) Coffea Arabica (Coffee) Seedcake Extract (Ecoffea) | 4% |
| | |

| Composition 13 | |
|---|---|
| SkQ1 | 0.1-1,000 mcM |
| Biocol LG | 2,9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| Sorbitol (and) Methylchromone (RonaCareLuremin) | 3% |

| Composition 14 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| Bismuth Oxychloride (RonaFlair MTU) | 2% |

| Composition 15 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| Mica (and) Titanium Dioxide (and) Iron Oxide (Colorona Red Gold) | 0.6% |

| Composition 16 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| Aqua (and) Acetyl Hexapeptide-8 (Argireline) | 5% |
| Microcare IT (methylchloroisothiazolinone (and) methylisothiazolinone) | 0.1% |

| Reference Composition 17 | (on basis of exp. of example 3) |
|---|---|
| SkQ1 | 0.1-1,000 mcM |
| Methocel 40-202 (hydroxypropyl methyl cellulose) | 2% |
| Propylene glycol | 3% |
| Pentylene glycol | 5% |
| Ethyl hexyl glycerine | 0.3% |
| Gatuline In-Tense | 1.8% |
| RonaCare Luremin | 3% |

| Reference Composition 18 | |
|---|---|
| SkQ1 | 0.1-1,000 mcM |
| Methocel 40-202 (hydroxypropyl methyl cellulose) | 2% |
| Propylene glycol | 3% |
| Pentylene glycol | 5% |
| Ethyl hexyl glycerine | 0.3% |
| Gatuline In-Tense | 1.8% |
| Ecoffea | 4% |

| Reference Composition 19 | |
|---|---|
| SkQ1 | 0.1-1,000 mcM |
| Methocel 40-202 (hydroxypropyl methyl cellulose) | 2% |
| Propylene glycol | 3% |
| Pentylene glycol | 5% |
| Ethyl hexyl glycerine | 0.3% |
| Argireline | 5% |
| RonaCare Luremin | 3% |
| | |

| Reference Composition 20 | |
|---|---|
| SkQ1 | 0.1-1,000 mcM |
| Methocel 40-202 (hydroxypropyl methyl cellulose) | 2% |
| Propylene glycol | 3% |
| Pentylene glycol | 5% |
| Ethyl hexyl glycerine | 0.3% |
| Argireline | 5% |
| Ecoffea | 4% |

| Reference Composition 21 | |
|---|---|
| SkQ1 | 0.1-1,000 mcM |
| Methocel 40-202 (hydroxypropyl methyl cellulose) | 2% |
| Propylene glycol | 3% |
| Pentylene glycol | 5% |
| Ethyl hexyl glycerine | 0.3% |
| Ecoffea | 4% |
| RonaCare Luremin | 3% |

| Reference Composition 22 | |
|---|---|
| SkQ1 | 0.1-1,000 mcM |
| Methocel 40-202 (hydroxypropyl methyl cellulose) | 2% |
| Caprylyl methicone (Silsoft 034) | 0.5% |
| Hydrogenated polydecene (Nexbase) | 0.5% |
| Propylene glycol | 3% |
| Pentylene glycol | 5% |
| Ethyl hexyl glycerine | 0.3% |
| Gatuline In-Tense | 1.8% |
| RonaCare Luremin | 3% |

| Reference Composition 23 | |
|---|---|
| SkQ1 | 0.1-1,000 mcM |
| Methocel 40-202 (hydroxypropyl methyl cellulose) | 2% |
| Caprylyl methicone (Silsoft 034) | 0.5% |
| Hydrogenated polydecene (Nexbase) | 0.5% |
| Propylene glycol | 3% |
| Pentylene glycol | 5% |
| Ethyl hexyl glycerine | 0.3% |
| Gatuline In-Tense | 1.8% |
| Ecoffea | 4% |

| Composition 24 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Gatuline In-Tense | 1.8% |
| RonaCare Luremin | 3% |

| Composition 25 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Gatuline In-Tense | 1.8% |
| Ecoffea | 4% |

| Composition 26 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Argireline | 5% |
| RonaCare Luremin | 3% |

| Composition 27 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Argireline | 5% |
| Ecoffea | 4% |

| Composition 28 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Ecoffea | 4% |
| RonaCare Luremin | 3% |

| Composition 29 | |
|---|---|
| SkQ1 | 0.1-1,000 mcM |
| Caprylyl methicone (Silsoft 034) | 0.5% |
| Hydrogenated polydecene (Nexbase) | 0.5% |
| Biocol LG | 2.9% |
| Propylene glycol | 3% |
| Pentylene glycol | 5% |
| Ethyl hexyl glycerine | 0.3% |
| Gatuline In-Tense | 1.8% |
| RonaCare Luremin | 3% |

| Composition 30 | |
|---|---|
| SkQ1 | 0.1-1,000 mcM |
| Caprylyl methicone (Silsoft 034) | 0.5% |
| Hydrogenated polydecene (Nexbase) | 0.5% |
| Biocol LG | 2.9% |
| Propylene glycol | 3% |
| Pentylene glycol | 5% |
| Ethyl hexyl glycerine | 0.3% |
| Gatuline In-Tense | 1.8% |
| Ecoffea | 4% |

| Composition 31 | |
|---|---|
| SkQ1 | 0.1-1,000 mcM |
| Caprylyl methicone (Silsoft 034) | 0.5% |
| Hydrogenated polydecene (Nexbase) | 0.5% |
| Biocol LG | 2.9% |
| Propylene glycol | 3% |
| Pentylene glycol | 5% |
| Ethyl hexyl glycerine | 0.3% |
| Argireline | 5% |
| RonaCare Luremin | 3% |

| Composition 32 | |
|---|---|
| SkQ1 | 0.1-1,000 mcM |
| Caprylyl methicone (Silsoft 034) | 0.5% |
| Hydrogenated polydecene (Nexbase) | 0.5% |
| Biocol LG | 2.9% |
| Propylene glycol | 3% |
| Pentylene glycol | 5% |
| Ethyl hexyl glycerine | 0.3% |
| Argireline | 5% |
| Ecoffea | 4% |

| Composition 33 | |
|---|---|
| SkQ1 | 0.1-1,000 mcM |
| Caprylyl methicone (Silsoft 034) | 0.5% |
| Hydrogenated polydecene (Nexbase) | 0.5% |
| Biocol LG | 2.9% |
| Propylene glycol | 3% |
| Pentylene glycol | 5% |
| Ethyl hexyl glycerine | 0.3% |
| RonaCare Luremin | 3% |
| Ecoffea | 4% |

| Reference Composition 34 | |
|---|---|
| SkQ1 | 0.1-1,000 mcM |
| Methocel 40-202 (hydroxypropyl methyl cellulose) | 2% |
| Propylene glycol | 3% |
| Pentylene glycol | 5% |
| Ethyl hexyl glycerine | 0.3% |
| Argireline | 5% |
| RonaCare Luremin | 3% |
| Ecoffea | 4% |

| Reference Composition 35 | |
|---|---|
| SkQ1 | 0.1-1,000 mcM |
| Methocel 40-202 (hydroxypropyl methyl cellulose) | 0.2% |
| Propylene glycol | 3% |
| Pentylene glycol | 5% |
| Ethyl hexyl glycerine | 0.3% |

| Reference Composition 36 | (on basis of exp. of example 4) |
|---|---|
| SkQ1 | 0.1-1,000 mcM |
| Methocel 40-202 (hydroxypropyl methyl cellulose) | 2% |
| Propylene glycol | 3% |
| Pentylene glycol | 5% |
| Ethyl hexyl glycerine | 0.3% |

| Composition 37 | |
|---|---|
| SkQ1 | 0.1-1,000 mcM |
| Biocol LG | 2.5% |
| Propylene glycol | 3% |
| Pentylene glycol | 5% |
| Ethyl hexyl glycerine | 0.3% |

| Composition 38 | |
|---|---|
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Caprylyl methicone (Silsoft 034) | 0.5% |
| Hydrogenated polydecene (Nexbase) | 0.5% |
| | |

| **Additional compositions on basis of exp. of example 1** | |
|---|---|
| Composition 39 | |
| SkQ1 | 0.1-1,000 mcM |
| Biocol LG | 2.9% |
| Propylene glycol | 3% |
| Pentylene glycol | 5% |
| Ethyl hexyl glycerine | 0.3% |
| Dipotassium glycyrrhizate | 1% |

| Reference Composition 40 | |
|---|---|
| SkQ1 | 0.1-1,000 mcM |
| Sodium Polyacrylate Starch (Makimousse 12) | 1% |
| Propylene glycol | 3% |
| Pentylene glycol | 5% |
| Ethyl hexyl glycerine | 0.3% |

Other examples of compositions are compositions according to the table shown above, in which Biocol LG is replaced with Biocol AX. The experiment conducted showed that SkQ retrains its stability with such replacement.

Other examples of compositions are compositions containing 0.1-1,000 mcM of SkQ1 and Biocol of any type without emollients.

Another aspect of this disclosure is the method of manufacturing compositions containing a mitochondrial antioxidant. The said method includes the following procedures: addition to water of gel-forming agent (at temperature suitable for the gel-forming agent specifically chosen), mixing (for certain gel-forming agents after swelling, for certain gel-forming agents after the required pH value is reached), introduction of remaining active ingredients, in which case the oil-soluble active ingredients should preferably be introduced together with the hydrophobic emollients, and the mitochondrial antioxidant is introduced last.

### Experimental examples:

The experimental samples shown below illustrate possible ways of applying the invention.

All experiments concerning stability of the cosmetic components tested are conducted under a single plan. The composition was prepared by adding gel-forming agent to water at the corresponding temperature and pH. Depending on the type of gel-forming agent with mixing or withholding necessary to swell the component and/or achieve the specific pH values. Then, after mixing, the remaining components (oil-soluble active ingredients, together with hydrophobic emollients) and the mitochondrial antioxidant are added.

The mixture was then kept in darkness at different temperatures, and the mitochondrial antioxidant content levels were determined using the LCMS/MS method on a Waters TQD chromatographic mass spectrometer.

The quantitative analysis was conducted using the UPLC-MS/MS method with SkQ-d15 of a known concentration as the internal standard and 3-chlorperbenzoic acid as the oxidising agent. The analysis was conducted in accordance with the method shown in the PCS for Visomytin eye drops.

1 analytical sample was prepared from each sample (extraction of 1 ml of methanol 12 hours, thermoshaker (27°C. 1400 rpm), centrifuging, preparation of analytical samples with dilution 5 times).

Each standard sample was injected 6 times before every nine injections of analytical samples. S/Sstd for standard samples were averaged at 45 injections.

### Experimental example 1 (file Cos16-Cos29).

The basic composition (BC1) used in this experiment was a composition with the following ingredients:
Biocol LG - 2.9%
Propylene glycol - 3%
Pentylene glycol - 5%
Ethyl hexyl glycerine - 0.3%
SkQ1 - 2 mcM

The following versions of compositions were examined:

| **Composition number** | **Composition code** | **Ingredient** |
|---|---|---|
| **1** | **Cos16** | **BC1 + benzalkonium chloride 0.01%** |
| **2** | **Cos17** | **BC1 + hydrogenated polydecene (Nexbase) 1%** |
| **3** | **Cos18** | **BC1 + caprylyl methicone (Silsoft 034) 1%** |
| **4** | **Cos19** | **BC1 + ethyl hexyl olivate (Sensolene) 1 %** |
| **5** | **Cos20** | **BC1 + D-Panthenol 0.75%** |
| **6** | **Cos21** | **BC1 + neopentylglycol diheptanoate (Lexfeel 7) 1%** |
| **7** | **Cos22** | **BC1 + inulin in mixture with a-glucane oligosaccharide (Biolin/P) 5%** |
| **8** | **Cos23** | **BC1 + Sorbitol in mixture with yeast extract (Drieline) 1%** |
| **9** | **Cos24** | **BC1 + ethyl ascorbic acid 0.1%** |
| **10** | **Cos25** | **BC1 + dipotassium glycyrrhizate 1%** |
| **11** | **Cos26** | **BC1 + allantoin 0.3%** |
| **12** | **Cos27** | **BC1 + ethyl ascorbic acid 0.5%** |
| **13** | **Cos28** | **BC1 + sodium ascorbylphosphate 0.5%** |
| **14*** | **Cos29** | **Sodium Polyacrylate Starch (Makimousse 12) 1% + propylene glycol 3% + pentylene glycol 5% + ethyl hexyl glycerine 0.3% + SkQ1** |

| | | |
|---|---|---|
| * Reference | | |

**0-point:** Samples of cosmetics after manufacture and weighing were stored at -78..-80°C in dark plastic bottles 2 ml in volume until the analysis was conducted.

Time points: **Samples of cosmetics after manufacture and weighing were stored for a set number of months at 37°C in dark plastic bottles 2 ml in volume then stored at -78..-80°C until the analysis was conducted.**

**Test preparation:** Heating at room temperature (30 min), extraction with methanol (1 ml, vortex, thermoshaker 1.5 hours 1,200 rpm), centrifuging (15 min 13,400 rpm)

**TABLE I: RAW DATA**

| | **Med** | **uG/mLAve** | **AveDev** | **AveDev%** | **m(cos), mg** | | **time, month** | **uG/g** | **AveDev** |
|---|---|---|---|---|---|---|---|---|---|
| **Cos16** | 0.0493 | **0.0499** | 0.0010 | 2.1% | **108.68** | | 0.0 | **0.459** | 0.009 |
| | 0.0378 | **0.0379** | 0.0002 | 0.6% | **104.37** | | 1.0 | **0.363** | 0.002 |
| | 0.0355 | **0.0358** | 0.0005 | 1.3% | **96.33** | | 2.0 | **0.371** | 0.005 |
| | 0.0311 | **0.0312** | 0.0004 | 1.1% | **92.17** | | 3.0 | **0.338** | 0.004 |
| | 0.0323 | **0.0326** | 0.0006 | 1.8% | **106** | | 4.0 | **0.308** | 0.006 |
| | 0.0307 | **0.0310** | 0.0004 | 1.4% | **105.35** | | 5.0 | **0.294** | 0.004 |
| | 0.0284 | **0.0285** | 0.0002 | 0.7% | **104.65** | | 6.0 | **0.272** | 0.002 |
| | 0.0265 | **0.0265** | 0.0002 | 0.7% | **93.57** | | 7.0 | **0.283** | 0.002 |
| | 0.0247 | **0.0247** | 0.0001 | 0.6% | **91.99** | | 8.0 | **0.269** | 0.002 |
| **Cos17** | 0.0225 | **0.0228** | 0.0004 | 1.6% | **91.58** | | 0.0 | **0.249** | 0.004 |
| | 0.0214 | **0.0215** | 0.0003 | 1.5% | **105.19** | | 1.0 | **0.204** | 0.003 |
| | 0.0214 | **0.0213** | 0.0002 | 0.9% | **110.66** | | 2.0 | **0.193** | 0.002 |
| | 0.0154 | **0.0154** | 0.0000 | 0.0% | **110.39** | | 3.0 | **0.139** | 0.000 |
| | 0.0167 | **0.0166** | 0.0003 | 1.6% | **96.15** | | 4.0 | **0.172** | 0.003 |
| | 0.0143 | **0.0143** | 0.0001 | 0.7% | **91.22** | | 5.0 | **0.156** | 0.001 |
| | 0.0139 | **0.0140** | 0.0001 | 0.8% | **109.17** | | 6.0 | **0.128** | 0.001 |
| | 0.0138 | **0.0140** | 0.0005 | 3.3% | **111.5** | | 7.0 | **0.125** | 0.004 |
| | 0.0143 | **0.0142** | 0.0002 | 1.7% | **108.9** | | 8.0 | **0.130** | 0.002 |
| **Cos18** | 0.0372 | **0.0374** | 0.0007 | 2.0% | **96.12** | | 0.0 | **0.389** | 0.008 |
| | 0.0316 | **0.0314** | 0.0008 | 2.4% | **108.39** | | 1.0 | **0.290** | 0.007 |
| | 0.0232 | **0.0232** | 0.0002 | 0.9% | **101.55** | | 2.0 | **0.229** | 0.002 |
| | 0.0208 | **0.0208** | 0.0001 | 0.5% | **97.72** | | 3.0 | **0.213** | 0.001 |
| | 0.0186 | **0.0186** | 0.0002 | 0.9% | **100.54** | | 4.0 | **0.185** | 0.002 |
| | 0.0174 | **0.0174** | 0.0004 | 2.3% | **101.44** | | 5.0 | **0.172** | 0.004 |
| | 0.0169 | **0.0168** | 0.0004 | 2.1% | **104.65** | | 6.0 | **0.161** | 0.003 |
| | 0.0188 | **0.0185** | 0.0005 | 2.7% | **102.84** | | 7.0 | **0.179** | 0.005 |
| | 0.0179 | **0.0182** | 0.0006 | 3.4% | **110.35** | | 8.0 | **0.165** | 0.006 |
| **Cos19** | 0.0381 | **0.0379** | 0.0004 | 1.0% | **106.13** | | 00 | **0.358** | 0.003 |
| | 0.0353 | **0.0352** | 0.0005 | 1.4% | **105.31** | | 1.0 | **0.335** | 0.005 |
| | 0.0223 | **0.0223** | 0.0002 | 0.8% | **94.35** | | 2.0 | **0.236** | 0.002 |
| | 0.0223 | **0.0221** | 0.0004 | 1.8% | **97.05** | | 3.0 | **0.227** | 0.004 |
| | 0.0041 | **0.0041** | 0.0001 | 2.0% | **110.82** | | 4.0 | **0.037** | 0.001 |
| | 0.0150 | **0.0150** | 0.0000 | 0.2% | **100.39** | | 5.0 | **0.149** | 0.000 |
| | 0.0045 | **0.0046** | 0.0000 | 11% | **99.97** | | 6.0 | **0.046** | 0.000 |
| | 0.0096 | **0.0097** | 0.0002 | 1.6% | **103.46** | | 7.0 | **0.093** | 0.002 |
| | 0.0126 | **0.0126** | 0.0003 | 2.1% | **99.27** | | 8.0 | **0.127** | 0.003 |
| **Cos20** | 0.0374 | **0.0373** | 0.0004 | 1.1% | **110.4** | | 0.0 | **0.338** | 0.004 |
| | 0.0307 | **0.0308** | 0.0002 | 0.6% | **91** | | 1.0 | **0.339** | 0.002 |
| | 0.0238 | **0.0237** | 0.0004 | 1.6% | **97.83** | | 2.0 | **0.242** | 0.004 |
| | 0.0348 | **0.0349** | 0.0003 | 0.7% | **95.19** | | 3.0 | **0.366** | 0.003 |
| | 0.0223 | **0.0223** | 0.0003 | 1.1% | **114.08** | | 4.0 | **0.195** | 0.002 |
| | 0.0228 | **0.0228** | 0.0002 | 1.1% | **101.98** | | 5.0 | **0.224** | 0.002 |
| | 0.0174 | **0.0173** | 0.0001 | 0.6% | **106.8** | | 6.0 | **0.162** | 0.001 |
| | 0.0191 | **0.0191** | 0.0002 | 0.9% | **96.46** | | 7.0 | **0.198** | 0.002 |
| | 0.0178 | **0.0179** | 0.0001 | 0.7% | **102.84** | | 8.0 | **0.174** | 0.001 |
| **Cos21** | 0.0430 | **0.0429** | 0.0009 | 2.1% | **103.54** | | 0.0 | **0.414** | 0.009 |
| | 0.0418 | **0.0416** | 0.0005 | 1.3% | **92.57** | | 1.0 | **0.450** | 0.006 |
| | 0.0354 | **0.0353** | 0.0003 | 0.8% | **101.06** | | 2.0 | **0.349** | 0.003 |
| | 0.0454 | **0.0456** | 0.0008 | 1.7% | **106.21** | | 3.0 | **0.430** | 0.007 |
| | 0.0362 | **0.0364** | 0.0006 | 1.7% | **97.27** | | 4.0 | **0.374** | 0.006 |
| | 0.0431 | **0.0433** | 0.0004 | 1.0% | **110.68** | | 5.0 | **0.391** | 0.004 |
| | 0.0322 | **0.0321** | 0.0006 | 1.9% | **108.25** | | 6.0 | **0.296** | 0.006 |
| | 0.0339 | **0.0340** | 0.0002 | 0.7% | **90.59** | | 7.0 | **0.375** | 0.002 |
| | 0.0245 | **0.0243** | 0.0005 | 1.9% | **98.83** | | 8.0 | **0.246** | 0.005 |
| **Cos22** | 0.0372 | **0.0372** | 0.0007 | 1.8% | **112.87** | | 0.0 | **0.330** | 0.006 |
| | 0.0203 | **0.0203** | 0.0000 | 0.0% | **105.39** | | 1.0 | **0.193** | 0.000 |
| | 0.0147 | **0.0147** | 0.0000 | 0.0% | **94** | | 2.0 | **0.157** | 0.000 |
| | 0.0139 | **0.0138** | 0.0002 | 1.6% | **111.08** | | 3.0 | **0.124** | 0.002 |
| | 0.0087 | **0.0086** | 0.0001 | 1.5% | **102.66** | | 4.0 | **0.084** | 0.001 |
| | 0.0083 | **0.0083** | 0.0000 | 0.1% | **97.32** | | 5.0 | **0.085** | 0.000 |
| | 0.0060 | **0.0061** | 0.0001 | 1.7% | **97.72** | | 6.0 | **0.062** | 0.001 |
| | 0.0059 | **0.0059** | 0.0001 | 1.4% | **93.14** | | 7.0 | **0.063** | 0.001 |
| | 0.0063 | **0.0063** | 0.0000 | 0.3% | **98.38** | | 8.0 | **0.064** | 0.000 |
| **Cos23** | 0.0481 | **0.0480** | 0.0000 | 0.1% | **104.9** | | 0.0 | **0.458** | 0.000 |
| | 0.0279 | **0.0277** | 0.0004 | 1.3% | **109.68** | | 1.0 | **0.253** | 0.003 |
| | 0.0173 | **0.0174** | 0.0005 | 2.6% | **87.64** | | 2.0 | **0.199** | 0.005 |
| | 0.0186 | **0.0188** | 0.0004 | 2.0% | **89.39** | | 3.0 | **0.210** | 0.004 |
| | 0.0196 | **0.0198** | 0.0004 | 1.9% | **107.85** | | 4.0 | **0.184** | 0.003 |
| | 0.0138 | **0.0139** | 0.0002 | 1.4% | **94.48** | | 5.0 | **0.147** | 0.002 |
| | 0.0102 | **0.0102** | 0.0001 | 1.4% | **91.4** | | 6.0 | **0.112** | 0.002 |
| | 0.0125 | **0.0126** | 0.0004 | 2.8% | **95.03** | | 7.0 | **0.133** | 0.004 |
| | 0.0133 | **0.0134** | 0.0002 | 1.2% | **107.99** | | 8.0 | **0.124** | 0.001 |
| **Cos24** | 0.0393 | **0.0387** | 0.0010 | 2.7% | **97.47** | | 0.0 | **0.397** | 0.011 |
| | 0.0197 | **0.0196** | 0.0003 | 1.5% | **111.7** | | 1.0 | **0.175** | 0.003 |
| | 0.0085 | **0.0085** | 0.0001 | 1.5% | **103.49** | | 2.0 | **0.082** | 0.001 |
| | 0.0153 | **0.0153** | 0.0003 | 2.0% | **112.53** | | 3.0 | **0.136** | 0.003 |
| | 0.0116 | **0.0116** | 0.0001 | 0.5% | **93.66** | | 4.0 | **0.124** | 0.001 |
| | 0.0096 | **0.0095** | 0.0001 | 1.0% | **111.19** | | 5.0 | **0.086** | 0.001 |
| | 0.0050 | **0.0050** | 0.0001 | 1.2% | **99.02** | | 6.0 | **0.051** | 0.001 |
| | 0.0065 | **0.0065** | 0.0001 | 1.4% | **111** | | 7.0 | **0.059** | 0.001 |
| | 0.0053 | **0.0053** | 0.0001 | 2.8% | **91.19** | | 8.0 | **0.058** | 0.002 |
| **Cos25** | 0.0409 | **0.0405** | 0.0007 | 1.8% | **89.09** | | 0.0 | **0.454** | 0.008 |
| | 0.0311 | **0.0307** | 0.0005 | 1.6% | **109.77** | | 1.0 | **0.280** | 0.004 |
| | 0.0282 | **0.0279** | 0.0006 | 2.2% | **107.22** | | 2.0 | **0.260** | 0.006 |
| | 0.0239 | **0.0239** | 0.0002 | 0.9% | **93.9** | | 3.0 | **0.254** | 0.002 |
| | 0.0230 | **0.0231** | 0.0001 | 0.6% | **98.16** | | 4.0 | **0.235** | 0.001 |
| | 0.0226 | **0.0226** | 0.0005 | 2.2% | **110.1** | | 5.0 | **0.205** | 0.004 |
| | 0.0199 | **0.0198** | 0.0003 | 1.5% | **100.61** | | 6.0 | **0.196** | 0.003 |
| | 0.0204 | **0.0205** | 0.0003 | 1.3% | **111.4** | | 7.0 | **0.184** | 0.002 |
| | 0.0190 | **0.0191** | 0.0002 | 1.1% | **108.37** | | 8.0 | **0.177** | 0.002 |
| **Cos26** | 0.0446 | **0.0448** | 0.0005 | 1.0% | **104.55** | | 0.0 | **0.429** | 0.004 |
| | 0.0226 | **0.0227** | 0.0002 | 0.8% | **99.17** | | 1.0 | **0.229** | 0.002 |
| | 0.0188 | **0.0187** | 0.0002 | 1.2% | **103.84** | | 2.0 | **0.180** | 0.002 |
| | 0.0171 | **0.0172** | 0.0002 | 0.9% | **113.75** | | 3.0 | **0.151** | 0.001 |
| | 0.0110 | **0.0109** | 0.0002 | 2.2% | **89.97** | | 4.0 | **0.121** | 0.003 |
| | 0.0106 | **0.0104** | 0.0002 | 2.2% | **103.9** | | 5.0 | **0.100** | 0.002 |
| | 0.0078 | **0.0078** | 0.0001 | 1.6% | **100.73** | | 6.0 | **0.078** | 0.001 |
| | 0.0080 | **0.0080** | 0.0000 | 0.0% | **111.11** | | 7.0 | **0.072** | 0.000 |
| | 0.0059 | **0.0059** | 0.0000 | 0.0% | **105.86** | | 8.0 | **0.056** | 0.000 |
| **Cos27** | 0.0394 | **0.0404** | 0.0014 | 3.5% | **95.99** | | 0.0 | **0.421** | 0.015 |
| | 0.0237 | **0.0235** | 0.0003 | 1.2% | **100.15** | | 1.0 | **0.235** | 0.003 |
| | 0.01 59 | **0.0159** | 0.0003 | 2.1% | **94.61** | | 2.0 | **0.168** | 0.003 |
| | 0.0123 | **0.0121** | 0.0004 | 3.2% | **96.31** | | 3.0 | **0.125** | 0.004 |
| | 0.0100 | **0.0101** | 0.0004 | 3.8% | **109.65** | | 4.0 | **0.092** | 0.004 |
| | 0.0055 | **0.0055** | 0.0001 | 1.5% | **104.03** | | 5.0 | **0.053** | 0.001 |
| | 0.0031 | **0.0032** | 0.0001 | 3.4% | **111.45** | | 6.0 | **0.028** | 0.001 |
| | 0.0035 | **0.0034** | 0.0002 | 4.6% | **112.73** | | 7.0 | **0.030** | 0.001 |
| | 0.0025 | **0.0025** | 0.0000 | 0.0% | **105.36** | | 8.0 | **0.024** | 0.000 |
| **Cos28** | 0.0454 | **0.0457** | 0.0005 | 1.1% | **114.27** | | 0.0 | **0.400** | 0.004 |
| | 0.0091 | **0.0091** | 0.0001 | 0.7% | **95.23** | | 1.0 | **0.096** | 0.001 |
| | 0.0050 | **0.0050** | 0.0000 | 0.6% | **107.99** | | 2.0 | **0.047** | 0.000 |
| | 0.0026 | **0.0026** | 0.0001 | 3.6% | **95.14** | | 3.0 | **0.027** | 0.001 |
| | 0.0020 | **0.0020** | 0.0000 | 0.9% | **97.41** | | 4.0 | **0.021** | 0.000 |
| | 00017 | **0.0016** | 0.0001 | 3.7% | **95.45** | | 5.0 | **0.017** | 0.001 |
| | 00012 | **0.0012** | 0.0001 | 5.3% | **104.59** | | 6.0 | **0.011** | 0.001 |
| | 00010 | **0.0010** | 0.0000 | 31% | **92.12** | | 7.0 | **0.011** | 0.000 |
| | 0.0013 | **0.0013** | 0.0001 | 5.6% | **108.81** | | 8.0 | **0.012** | 0.001 |
| **Cos29*** | 0.0091 | **0.0091** | 0.0001 | 1.1% | **106.2** | | 0.0 | **0.085** | 0.001 |
| | 0.0084 | **0.0083** | 0.0001 | 1.4% | **103.75** | | 1.0 | **0.080** | 0.001 |
| | 0.0063 | **0.0065** | 0.0003 | 4.4% | **98.44** | | 2.0 | **0.066** | 0.003 |
| | 0.0055 | **0.0055** | 0.0001 | 1.5% | **87.85** | | 3.0 | **0.063** | 0.001 |
| | 0.0052 | **0.0051** | 0.0001 | 1.2% | **104.62** | | 4.0 | **0.049** | 0.001 |
| | 0.0051 | **0.0051** | 0.0001 | 1.2% | **102.09** | | 5.0 | **0.050** | 0.001 |
| | 0.0044 | **0.0044** | 0.0001 | 2.3% | **95** | | 6.0 | **0.047** | 0.001 |
| | 0.0044 | **0.0044** | 0.0001 | 1.5% | **98.51** | | 7.0 | **0.044** | 0.001 |
| | 0.0052 | **0.0053** | 0.0001 | 1.5% | **103.27** | | 8.0 | **0.051** | 0.001 |

| | | | | | | | | | |
|---|---|---|---|---|---|---|---|---|---|
| * Reference | | | | | | | | | |

On the basis of the kinetic curves and initial losses, the time taken to reach 20% losses, and residual SkQ1 content after 1 year of storage, were calculated. The values obtained were extrapolated to real conditions of storage at room temperature (25C) and in refrigeration temperature (4C). The values calculated are shown in the TABLE II

**TABLE II: EXTRAPOLATED DATA**

| Sample number | Degradation speed constant at 37°C k', month⁻¹ | Time taken to reach 80% of SkQ1 from added total, months | | | Residual SkQ1 content after year of storage, % | | |
|---|---|---|---|---|---|---|---|
| | | 37°C | 25 °C | 4 °C | 37 °C | 25 °C | 4°C |
| 1 | 0.0607 | 3.7 | 8.4 | 36.2 | 48 | 73 | 93 |
| 2 ^{∗} | 0.0796 | | | | 21 | 36 | 49 |
| 3 | 0.0966 | 0.5 | 1.2 | 5.0 | 27 | 51 | 75 |
| 4 ^{∗} | 0.1947 | | | | 8 | 28 | 62 |
| 5 ^{∗} | 0.0927 | | | | 24 | 45 | 66 |
| 6 | 0.0509 | 2.1 | 4.9 | 20.8 | 49 | 69 | 85 |
| 7 ^{∗} | 0.2026 | | | | 6 | 25 | 56 |
| 8 | 0.1444 | 1.5 | 3.5 | 15.0 | 18 | 47 | 84 |
| 9 | 0.2061 | 0.3 | 0.7 | 3.2 | 7 | 29 | 67 |
| 10 | 0.0968 | 2.2 | 5.0 | 21.3 | 31 | 60 | 88 |
| 11 | 0.2283 | 0.6 | 1.5 | 6.2 | 6 | 28 | 71 |
| 12 | 0.3679 | 0.3 | 0.8 | 3.3 | 1 | 13 | 59 |
| 13 | 0.3982 | 0.2 | 0.4 | 1.8 | 1 | 11 | 54 |
| 14 ^{∗}(^{∗∗}) | 0.0791 | | | | 7 | 12 | 17 |

| | | | | | | | |
|---|---|---|---|---|---|---|---|
| * - samples in which initial reduction of concentration of SkQ1 exceeds 20%. (**) Reference | | | | | | | |

Conclusion: Compositions 1 and 6 provide greater SkQ1 stability levels.

## Claims

1. A composition comprising a mitochondrial antioxidant SkQ1: 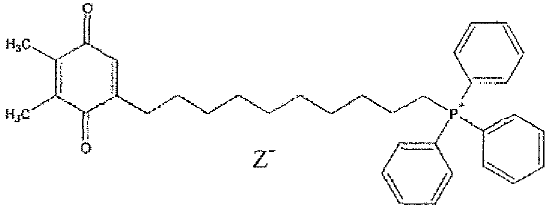 or its reduced form,
where Z⁻ is a pharmacologically acceptable anion,
and ceratonia siliqua gum, algin, pectin, ethyl hexyl glycerine, pentylene glycol, and propylene glycol.

2. A cosmetic composition consisting of a composition according to claim 1 and a further compound selected from the group consisting of benzalkonium chloride, hydrogenated polydecene, caprylyl methicone, ethyl hexyl olivate, D-Panthenol, neopentylglycol diheptanoate, inulin in mixture with α-glucane oligosaccharide, ethyl ascorbic acid, dipotassium glycyrrhizate, allantoin, and sodium ascorbylphosphate.

## Patentansprüche

1. Zusammensetzung, umfassend ein mitochondriales Antioxidans SkQ1: 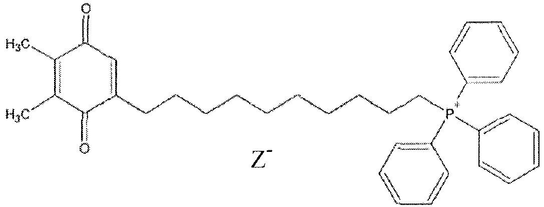 oder dessen reduzierte Form,
worin Z⁻ ein pharmakologisch annehmbares Anion ist,
und Johannisbrotbaum-Gummi, Algin, Pektin, Ethylhexylglycerin, Pentylenglycol und Propylenglycol.

2. Kosmetische Zusammensetzung bestehend aus einer Zusammensetzung gemäß Anspruch und einer weiteren Verbindung, ausgewählt aus der Gruppe, bestehend aus Benzalkoniumchlorid, hydriertem Polydecen, Caprylylmethicon, Ethylhexylolivat, D-Panthenol, Neopentylglycoldiheptanoat, Inulin im Gemisch mit α-Glucanoligosaccharid, Ethylascorbinsäure, Dikaliumglycyrrhizat, Allantoin und Natriumascorbylphosphat.

## Revendications

1. Composition, comprenant un antioxydant mitochondrial SkQ1: 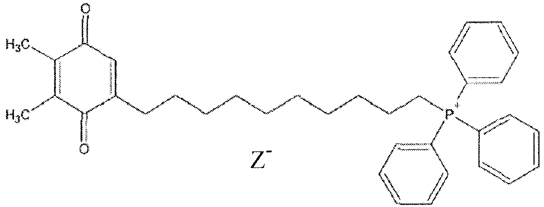 ou sa forme réduite,
dans laquelle Z⁻ est un anion pharmacologiquement acceptable, et de la gomme de ceratonia siliqua, de l'algine, de la pectine, de l'éthylhexylglycérine, du pentylène glycol et du propylène glycol.

2. Composition cosmétique consistant en une composition selon la revendication et un autre composé choisi parmi le groupe constitué du chlorure de benzalkonium, le polydécène hydrogéné, le caprylyl methicone, l'olivate d'éthylhexyle, D-panthénol, l'heptanoate de néopentylglycol, l'inuline dans un mélange avec un α-inuline-oligosaccharide, l'ester éthylique de l'acide ascorbique, l'allantoïne et l'ascorbylphosphate de sodium.
